# EUROPEAN PATENT SPECIFICATION

(11) **EP 3 581 659 B1**
(45) Date of publication and mention of the grant of the patent: **04.11.2026**
(21) Application number: 18183574.5
(22) Date of filing: 15.07.2018
(51) Int. Cl.: C12P 39/00, C12P 3/00, C12P 7/42

(54) **METHOD FOR ONE-POT CO-PRODUCTION OF CAPROIC ACID AND HYDROGEN**
VERFAHREN ZUR EINTOPF-CO-PRODUKTION VON CAPRONSÄURE UND WASSERSTOFF
PROCÉDÉ DE CO-PRODUCTION À UN POT D'ACIDE CAPROIQUE ET D'HYDROGÈNE

(30) Priority: 15.06.2018 PL 42594818
(43) Date of publication of application: 18.12.2019
(73) Proprietor: Politechnika Poznanska, 60-965 Poznan (PL)
(72) Inventor: Oleskowicz-Popiel, Piotr, 61-245 Poznan (PL); Zagrodnik, Roman, 61-690 Poznan (PL); Duber, Anna, 61-212 Poznan (PL)
(74) Representative: Tyrala, Magdalena

(56) References cited:
- P YANG ET AL: "Biohydrogen production from cheese processing wastewater by anaerobic fermentation using mixed microbial communities", INTERNATIONAL JOURNAL OF HYDROGEN ENERGY., vol. 32, no. 18, 1 December 2007 (2007-12-01), GB, pages 4761 - 4771, XP055559395, ISSN: 0360-3199, DOI: 10.1016/j.ijhydene.2007.07.038
- JANKOWSKA EWELINA ET AL: "Volatile fatty acids production during mixed culture fermentation - The impact of substrate complexity and pH", CHEMICAL ENGINEERING JOURNAL, ELSEVIER, AMSTERDAM, NL, vol. 326, 7 June 2017 (2017-06-07), pages 901 - 910, XP085129055, ISSN: 1385-8947, DOI: 10.1016/J.CEJ.2017.06.021
- STEPHEN J. ANDERSEN ET AL: "Electrolytic extraction drives volatile fatty acid chain elongation through lactic acid and replaces chemical pH control in thin stillage fermentation", BIOTECHNOLOGY FOR BIOFUELS, vol. 8, no. 1, 1 December 2015 (2015-12-01), XP055558411, DOI: 10.1186/s13068-015-0396-7
- KUCEK LEO A ET AL: "Conversion of l-lactate into n-caproate by a continuously fed reactor microbiome", WATER RESEARCH, ELSEVIER, AMSTERDAM, NL, vol. 93, 15 February 2016 (2016-02-15), pages 163 - 171, XP029442284, ISSN: 0043-1354, DOI: 10.1016/J.WATRES.2016.02.018
- XIAOYU ZHU ET AL: "The synthesis of n-caproate from lactate: a new efficient process for medium-chain carboxylates production", SCIENTIFIC REPORTS, vol. 5, no. 1, 25 September 2015 (2015-09-25), XP055558125, DOI: 10.1038/srep14360
- JIAJIE XU, ET AL.,: "Temperature-Phased Conversion of Acid Whey Waste Into Medium-Chain Carboxylic Acids via Lactic Acid: No External e-Donor", JOULE, vol. 2, no. 2, 21 February 2018 (2018-02-21), pages 280 - 295, XP009511360, DOI: 10.1016/j.joule.2017.11.008
- HONG-BO DING ET AL: "Caproate formation in mixed-culture fermentative hydrogen production", BIORESOURCE TECHNOLOGY, vol. 101, no. 24, 1 December 2010 (2010-12-01), AMSTERDAM, NL, pages 9550 - 9559, XP055558130, ISSN: 0960-8524, DOI: 10.1016/j.biortech.2010.07.056
- JOANNA CHWIALKOWSKA ET AL: "Caproic acid production from acid whey via open culture fermentation - Evaluation of the role of electron donors and downstream processing", BIORESOURCE TECHNOLOGY, vol. 279, 19 January 2019 (2019-01-19), AMSTERDAM, NL, pages 74 - 83, XP055558284, ISSN: 0960-8524, DOI: 10.1016/j.biortech.2019.01.086
- ANNA DUBER ET AL: "Exploiting the real wastewater potential for resource recovery - n -caproate production from acid whey", GREEN CHEMISTRY, vol. 20, no. 16, 23 July 2018 (2018-07-23), GB, pages 3790 - 3803, XP055559382, ISSN: 1463-9262, DOI: 10.1039/C8GC01759J

## Description

The present invention relates to a one-step method for the co-production of caproic acid and hydrogen from an organic carbon source in the form of biomass.

### BACKGROUND

In light of increasing demand for energy and sustainable development the production of bioenergy and renewable chemicals is a must. Intense economic development and urbanization result in an inevitable increase in energy demand, which is also linked to the depletion of conventional resources sources such as crude oil, natural gas and other fossil fuels. The above trend focuses the world's attention to search for novel biotechnological solutions enabling efficient and sustainable development. The continuous increase in organic and biomass waste brings a great energy potential and it can make a substantial contribution to waste management development and supply in so-called green energy. Recently, waste streams have been regarded as renewable bioresources that can be valorised to value-added commodities, such as liquid biofuels, bioplastics or other biochemicals.

Carboxylate platform is one of the biorefinery platform where carbon and energy from organic feedstock, usually agricultural or industrial waste biomass, can be recovered through open culture fermentation (OCF). In OCF, similarly to anaerobic digestion, the raw feedstock is first homogenised by hydrolytic bacteria. In the subsequent step obtained intermediates undergo primary fermentation to economically low-value compounds, mostly short-chain carboxylic acids (SCCAs) that range from two to five carbons including acetate, propionate, n-butyrate and n-valerate. In the same fermentation process, they can be either used as precursors in the secondary fermentation step or directly extracted from the fermentation broth and then converted into industrially useful biochemicals in downstream processing. In the secondary fermentation step the direction of interest is elongation of SCCAs to medium-chain carboxylic acids (MCCAs) ranging from six to nine carbons.

Caproic acid, a carboxylate of six carbons, is one of the MCCAs that has gained more attention recently. Caproic acid has a wide range of applications such as feed additive, antimicrobial or plant growth promoter as well as a precursor to various commodities including fragrances, paint additives and pharmaceuticals. It is also used as a synthetic lubricant and refrigeration lubricant in production of metal working fluids to enhance rust resistance, cutting capabilities and grinding capabilities of fluids. The synthetic production is not well established. A shift towards bio-based products is observed with the aim to decrease carbon footprint and to reduce dominant dependence on petrochemicals. Caproic acid due to its high caloric value and slightly hydrophobic properties is a suitable intermediate for biofuels and biochemicals production. Consequently it could be further processed into biofuel.

In addition to soluble products, microbiological processes can also generate valuable gas products, such as hydrogen and methane. Hydrogen is favored over methane for two reasons. First of all, it has wider range of industrial applications as compared to methane. It can be used for the synthesis of alcohol, aldehydes and ammonia as well as hydrogenation of petroleum. Secondly, hydrogen is widely deemed as one of the most promising fuels of the future. It is clean energy carrier - produces water upon combustion, and in addition to use in internal combustion engines, it can also be used in fuel cells for production of electricity. Currently, about 96% of hydrogen comes from processes based on fossil fuels. Therefore, the development of clean and economic methods of hydrogen production is essential for hydrogen economy.

US8501463 describes anaerobic fermentation systems for production of chemicals such as hydrogen and mixed volatile organics acids. The main emphasis is placed on the construction of the fermentation apparatus. Liquid products consist primarily of short-chain organic compounds (C2-C5). US2010/0248318 also describes a method for production of mixed carboxylic acids from biomass. Hydrogen gas produced during fermentation is used for the production of mixed primary alcohols. It is focused on methods for removing impurities and cleaning product streams. Our invention relates to specific production of caproic acid (C6) and hydrogen as two key products and it is not focused on the other microbial products of the fermentation processes. EP2271764 relates to a method for enzymatic production of C6-C18 fatty alcohols or C8-C18 fatty acids from biomass, wherein the electron donor is hydrogen, ethanol, n-butanol and/or mixtures thereof. Similarly, US9650652 describes methods and systems for production of C6-C8 carboxylates from biomass with methane as gaseous co-product, wherein the electron donor is alcohol. Our invention relates to production of hydrogen and caproic acid (C6 carboxylate) with lactate as electron donor, which, conversely to the aforementioned methods, does not have to be added from an external substrate and it is generated within the system as an intermediate product. CN104357496 describes a multi-step process for production of caproic acid solely from lactic acid, while our invention relates to one-step process which can be carried out in one bioreactor, henceforth significantly reducing capital and operating costs. Furthermore, we developed a process where any organic substrate could be converted into caproic acid and hydrogen with lactate as an intermediate product and not a primary carbon source.

Initial studies on production of caproic and caprylic acids were presented by Steinbusch et al. [1]. This study presented elongation of acetate with ethanol and hydrogen as electron donors in batch experiments. Agler et al. [2] described similar process for continuous production of caproic acid and methane from ethanol containing waste. Nzeteu et al. [3] presented semi-continuous production of caproic acid from food waste with lactate as main electron donor and carbon source. However, no hydrogen was produced during this process. Moreover, lactate served as carbon source, whereas in our case it is an intermediate (*in-situ*) product. Zhu et al. [4] described caproic acid synthesis from lactic acid and ethanol containing waste. However, the process was performed in batch mode and substrate was boiled (sterilized) before process. We, on the other hand, indicated that it would be possible to convert unsterilized wastewaters into caproic acid and hydrogen in a continuous single-step bioprocess, which has several significant advantages over batch mode sterile bioprocess. Kucek et al. [5] reported caproic acid production from synthetic medium containing lactate and butyrate in continuous process (no hydrogen was produced). As a continuation, Xu et al. [6] utilized acid whey for production of C6-C9 carboxylates with lactate as intermediate, but two-stage process was required for this conversion. Caproic acid production according to our invention is performed in one-step process which is a major achievement. Consolidating bioprocesses into single step significantly reduces the investment and operational costs.

Document P. Yang et al. [7] discloses an anaerobic fermentation of cheese processing wastewater using mixed microbial communities for the purpose of biohydrogen production. In this process, hexanoic acid (caproic acid) is detected as one of the fermentation by-products. The described method, however, is characterized by a low maximum concentration of caproic acid, reaching only approximately 1.2 g/L. Furthermore, the process suffers from significant and unstable methane production, with methane content in the biogas often fluctuating and exceeding 15-30%. In contrast, the present invention describes a one-step process optimized for the co-production of both caproic acid and hydrogen. As demonstrated in the examples, the present invention achieves significantly higher caproic acid concentrations while simultaneously achieving effective and stable suppression of methanogenic activity (below 2% methane in the gaseous phase).

### REFERENCES

US 2010/0248318 - Granda et. al
US 8501463 B2 - Cox et al.
EP 2271764 B1 - Hamelers et al.
US 9650652 B2 - Angenent et. al
[1] Steinbusch KJJ, Hamelers HVM, Plugge CM, Buisman CJN. Biological formation of caproate and caprylate from acetate: fuel and chemical production from low grade biomass. Energy Environ Sci 2010;4:216-24.
[2] Agler MT, Spirito CM, Usack JG, Werner JJ, Angenent LT. Chain elongation with reactor microbiomes: upgrading dilute ethanol to medium-chain carboxylates. Energy Environ Sci 2012;5:8189-92.
[3] Nzeteu CO, Trego AC, Abram F, O'Flaherty V. Reproducible, high-yielding, biological caproate production from food waste using a single-phase anaerobic reactor system. Biotechnol Biofuels 2018;11:108.
[4] Zhu X, Tao Y, Liang C, Li X, Wei N, Zhang W, et al. The synthesis of n-caproate from lactate: a new efficient process for medium-chain carboxylates production. Sci Rep 2015;5:14360.
[5] Kucek LA, Nguyen M, Angenent LT. Conversion of l-lactate into n-caproate by a continuously fed reactor microbiome. Water Res 2016;93:163-71.
[6] Xu J, Hao J, Guzman JJL, Spirito CM, Harroff LA, Angenent LT. Temperature-Phased Conversion of Acid Whey Waste Into Medium-Chain Carboxylic Acids via Lactic Acid: No External e-Donor. Joule 2018;2:280-95.
[7] Yang P, Zhang R, McGarvey JA, Benemann JR. Biohydrogen production from cheese processing wastewater by anaerobic fermentation using mixed microbial communities. Int. J. Hydrog. Energy 2007; 32, 4761-4771.

### SUMMARY OF INVENTION

The invention provides a one-step method for the co-production of caproic acid and hydrogen as defined in claim 1. The dependent claims define preferred variants of the invention.

### DETAILED DESCRIPTION OF THE INVENTION

The present invention provides method for co-production of caproic acid and hydrogen from organic carbon source in the form of biomass substrate in a single bioreactor. The term "caproic acid" as used herein, refers to C6 carboxylic acid or carboxylic acid salt (caproate). Biological formation of caproic acid is driven by microbial consortia and until now the elongation process, called reversed β-oxidation, has been identified as the key reaction for caproic acid production. The reaction needs an electron donor, which, according to the invention, is lactic acid produced within the process itself as an intermediate product and/or delivered with the organic carbon source. However, most reports demonstrated the dependence on addition of external ethanol for efficient chain elongation, which limits range of application and increases the cost of synthesis. Thus, one of the possible secondary fermentation pathway to produce caproic acid may occur through the coupling of ethanol oxidation and n-butyrate reduction (ethanol + n-butyrate -> n-caproate). However, no relevant literature and patents have been published on simultaneous synthesis of caproic acid and hydrogen using lactic acid as an electron donor and an intermediate product.

Method according to the invention relies on a one-step co-production of caproic acid and hydrogen and comprises the following steps:
a) providing organic carbon source in the form of biomass and the open culture of microorganisms in a single bioreactor, wherein the open culture contains lactic acid producing consortia and consists of at least Coriobacteriaceae, Prevotellaceae, Clostridiaceae, Ruminococaceae and Acetobacteriaceae families,
b) transformation of organic carbon source in the presence of microorganisms, wherein lactic acid, which serves as an electron donor, is produced in-situ as an intermediate product and/or is present in the organic carbon source, and is further converted to caproic acid in the liquid phase and hydrogen in the gaseous phase, wherein the gaseous phase comprises less than 2% of methane and at least 20% of hydrogen;
c) separating the formed caproic acid and/or hydrogen.

Preferably the transformation of organic carbon source is carried out at 25-40°C, pH of from 4 to 8, organic loading rate between 3.6 and 108 g COD/L/day, and hydraulic retention time between 6 hours and 20 days.

The open culture of microorganisms are mixed natural consortia of microbes which originate from anaerobic digesters, acidogenic processes, activated sludge, intestinal microorganisms, rumen microorganisms, soil microorganisms, marine microorganisms. According to the invention, the open culture contains lactic acid producing consortia and consists of at least Coriobacteriaceae, Prevotellaceae, Clostridiaceae, Ruminococaceae and Acetobacteriaceae families.

It is noticeable that the products namely caproic acid and hydrogen are present in liquid and gaseous phase, whereas the gaseous phase includes hydrogen and carbon dioxide. One must also notice that methane formation is inhibited by the operating parameters, which according to the invention are set appropriately to obtain gaseous phase comprising less than 2% of methane. The method also turns out to be effective in the situation when the organic carbon source contains lactic acid.

The caproic acid may be separated by extraction, precipitation, flotation, sedimentation or adsorption.

Method according to the invention enables conversion of any non-sterile substrate of organic originate into two valuable co-products (caproic acid and hydrogen). Moreover, it has been proven that high productivity can be achieved in one step bioprocess which significantly simplify the value chain while reducing the capital and operating costs.

Suitable substrates for the process are various sources of carbohydrate containing biomass. Specific examples of suitable substrate include, but are not limited to: municipal waste and wastewater, agricultural waste and wastewater, waste and wastewater from food production, lignocellulosic biomass. Accordingly, this biomass will not compete with food production. Lactic acid, which serves as an electron donor, is produced in-situ as an intermediate product and/or is present in the organic carbon source. In one embodiment, the substrate is an acid whey. Whey is a byproduct of dairy industry, it is estimated that the yearly global production reaches 180-190 million tonnes. Acid whey, a by-product from making Greek yogurt, cottage and quark cheese, and alike, constitutes of serious environmental problem, but at the same time it has a potential of being transformed into an economically valuable products. The worldwide volume is expected to rise significantly in the coming years. It inherently contains lactose, lactate, minerals, peptides, vitamins and other minor components, thus can be used as a complex feedstock for the process.

Without intending to be bound by any particular theory it is considered that carbon source present in the biomass is converted to simple organic compounds (C2-C4), which are required during the synthesis as they act as an electron acceptors. Specific examples of suitable organic compound are: acetate, n-propionate, i-propionate, n-butyrate, i-butyrate. Lactic acid mixed consortia are responsible for production of electron donor in the form of lactic acid from the substrate. Further, lactic acid drives the reactions, which synthesize electron acceptors into caproic acid. Hydrogen is also formed as a co-product, as part of electrons is redirected to proton reduction.

The method according to the present invention is conducted in a single bioreactor, so that all above processes take place simultaneously. Various operational strategies could be applied including batch, fed-batch or continuous reactor processes. Considering process yield and process economics, the continuous processes are preferred.

The synthesis process of the present invention is an enzymatic process, which is carried out by microorganisms at anaerobic conditions. It is important to note that pure cultures of microorganisms are not necessary. In various embodiments biochemical conversion is carried out by open culture of microorganisms. The open culture microorganisms which are used in this invention can be obtained from a number of inoculum sources such as anaerobic digesters, acidogenic processes, activated sludge, intestinal microorganisms, rumen microbes, soil microorganisms, and marine microorganisms. In one embodiment open culture of microorganisms is supplemented with substrate containing lactic acid mixed consortia. Using reactor microbiomes for biological production of biochemicals such as caproic acid is advantageous as open cultures are resistant to possible disturbances that may occur during the process and, in contrast to using pure culture, no sterilization procedures are required prior the fermentation. The relative population of microorganisms is manipulated by proper control of operational parameters (pH, organic loading rate, hydraulic and sludge retention time, temperature).

Caproic acid and hydrogen production according to the invention is carried out at mesophilic conditions. A suitable temperature is within the range of 25 - 40 °C. It is preferred to maintain the pH within the range of 4-8. The process can be performed in a wide range of organic loading rates (OLR). In one embodiment the OLR can be from 3,6 - 108 g COD/L/day. The process can be performed in a wide range of HRT. In one embodiment the HRT can be from 6 h - 20 days.

The products formed in the described method are present in the gaseous phase and liquid phase. The liquid phase can contain, for example, C2, C4, C6 carboxylates and alcohols. In one embodiment specificity of caproic acid production reaches 82%. Due to long carbon chain, caproic acid is partially insoluble in the reaction broth. Therefore, it could be quite easily separated from medium. The methods of caproic acid separation include: extraction, precipitation, flotation, sedimentation or adsorption.

The gaseous component of the product can comprise hydrogen, methane, and carbon dioxide. As mentioned above methane is not a preferable product. Adding inhibitors can inhibit methane production, but it is expensive and not suitable for industrial applications. In the present invention there was no need for inhibition of methanogenesis by external inhibitors. Methane formation was inhibited with the operating parameters. To inhibit methanogenic activity and promote lactate to caproic acid conversion we evaluated the process under the regime of low pH (4-6) with decreasing hydraulic retention time (HRT). According to the invention methane gas concentration during the reaction is always less than 2%. The hydrogen is preferred gaseous product. According to the invention gaseous phase comprises of at least 20% of hydrogen.

The high concentrations of caproic acid could be toxic for the cells of microorganisms. The inhibitory effect of the final product makes it difficult to increase the concentration of caproic acid. Therefore, its higher concentrations inhibit the growth and activity of microorganisms Therefore, it is preferable to partially redirect the process for the production of hydrogen. Without wishing to be bound by any particular theory it is considered that redirecting part of electrons to hydrogen production may reduce caproic acid production rate, which may reduce its toxic effect on microorganisms.

The following examples are given to illustrate different embodiments of the invention, but are not intended to limit the methods according to the invention to any extent. The embodiments are supported by drawings where fig. 1 shows the concentration profile of carboxylates, ethanol and lactate along the fermentation of acid whey; fig. 2 caproic acid and total carboxylates production rate and caproic acid specificity; fig. 3 presents the concentration profile of carboxylates, ethanol and lactate along the fermentation of acid whey (where start-up phase is not shown) and finally fig. 4 shows the production rate of gaseous products along the fermentation of acid whey. Table 1 presents microbial composition of bioreactor samples on day 217 at family level - however the table contains only families that exceeded 1% relative abundance in bioreactor are presented. The families with less than 1% relative abundance are gathered and assigned as Other.

### Example 1.

Inoculum was originating from anaerobic digester sludge. Acid whey, the dairy industry wastewater after traditional quark production was used as a feedstock. Total chemical oxygen demand (COD) of whey was 71.9 g/L, while concentration of lactate and lactose were 25.2 g/L and 18.6 g/L, respectively. The experiment was carried out in an 1 L Upflow Anaerobic Sludge Blanket (UASB) reactor. The temperature inside the reactor was maintained at 30 °C by circulating heated water through a water jacket. The pH in the reactor was automatically adjusted to 5.5. Organic acids and alcohols concentration were monitored with gas chromatography (GC, Shimadzu, Japan), while concentrations of lactate and lactose were determined with high performance liquid chromatography (HPLC 20AT, Shimadzu, Japan). To characterize the microbial population metagenomic sequencing of V3-V4 hypervariable region of 16S rRNA gene was performed with Illumina Miseq v2 Reagent kit.

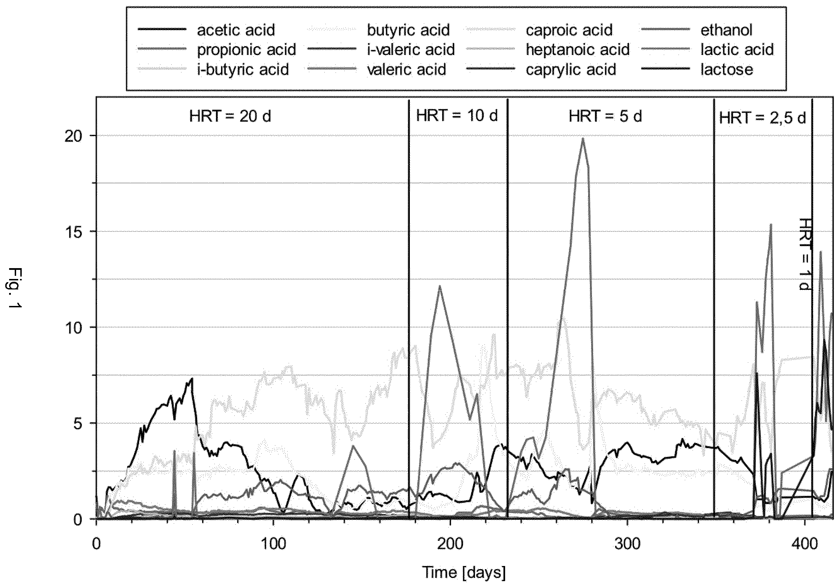

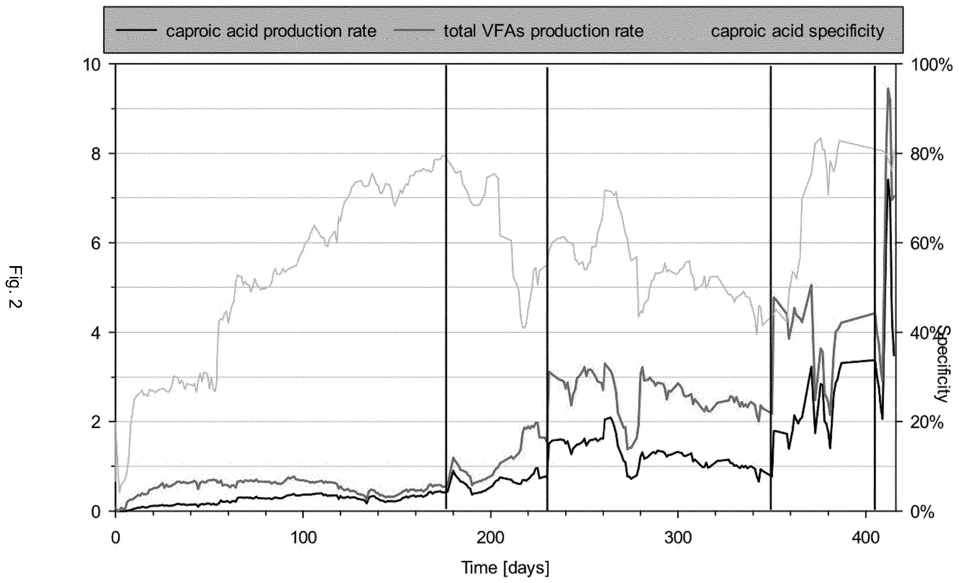

The reactor was operated for 415 days with decreasing HRT, where five subsequent operating stages were distinguished along the process. Start-up stage lasted from day 0 to 177. During that period the HRT was maintained at 20 days to let the development of caproic acid producing microbiome. Stable caproic acid production was observed after about 100 days with average caproic acid concentration of 7.0 g/L (fig. 1) Lactate was not accumulated during these days suggesting that the caproic acid production was linked with lactate utilization. Moreover, each decrease in caproic acid production resulted in an increase of lactate concentration. In that phase the caproic acid reached the maximum concentration of 8.9 g/L (day 173) with specificity between 68-77% (fig. 2).

Decrease of HRT to 10 days and then to 5 days increased caproic acid production to 0.7 g/L/day and 1.6 g/L/day, respectively. Caproic acid concentration of 10.5 g/L (and specificity of 67%) on day 264 was the highest concentration of caproic acid in the fermentation broth. HRT of 2.5 day resulted in significant increase in caproic acid production rate up to 3.3 g/L/day and specificity of over 70% during days 366 - 387 with maximum specificity of 79% on days 377 and 387. Shortening the HRT to 1 day was important for ultimate increase in caproic acid production rate that reached maximum of 7.4 g/L/day with specificity of 78%. The reactor was fed with acid whey, where most of the carbon was introduced as the carbon in lactose and lactic acid. Lactose can be fermented mostly to lactic acid, which suggested that lactate was not only delivered to the reactor with feed but also produced within the reactor. The predominance of lactate with simultaneous ethanol production in the reactor indicated that the caproic acid synthesis was performed primarily by lactate utilization and to a lesser extent through ethanol.

The structural biodiversity of the bioreactor microbiome was calculated as Shannon index (*H'*)*.* For the reactor samples *H'* counted of 4.3 on average, which indicates that the reactor microbiome was quite diverse but also stable, as the index did not vary much along the process. The Gini index was very high (0.98) over the operating period, proving that the reactor microbiome was highly specialized for caproic acid production. The example microbial composition with relative abundance of the bioreactor is shown in Table 1.

### Example 2.

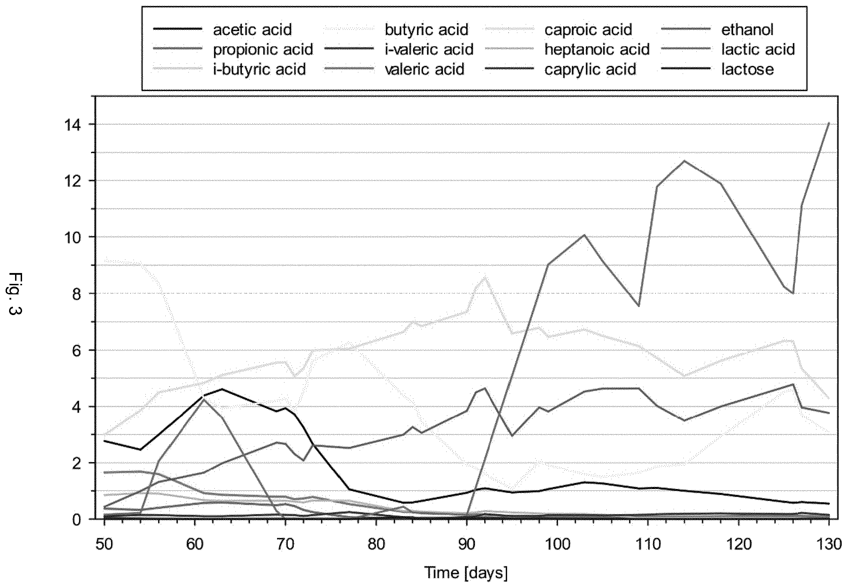

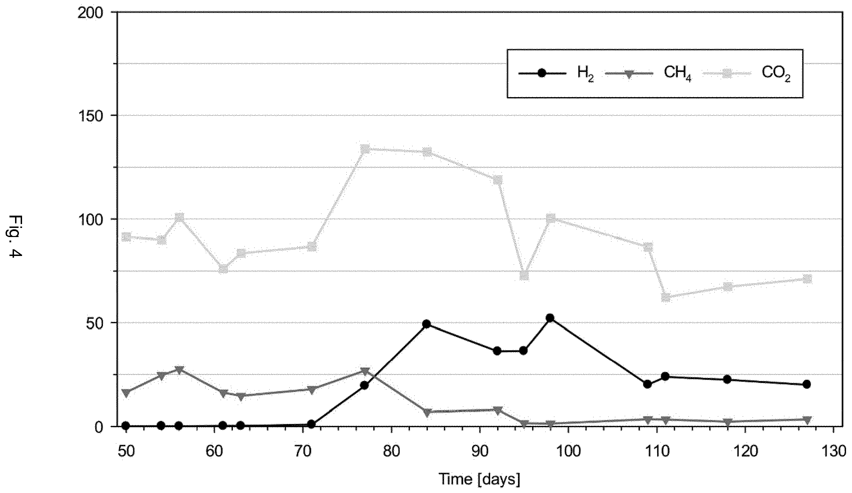

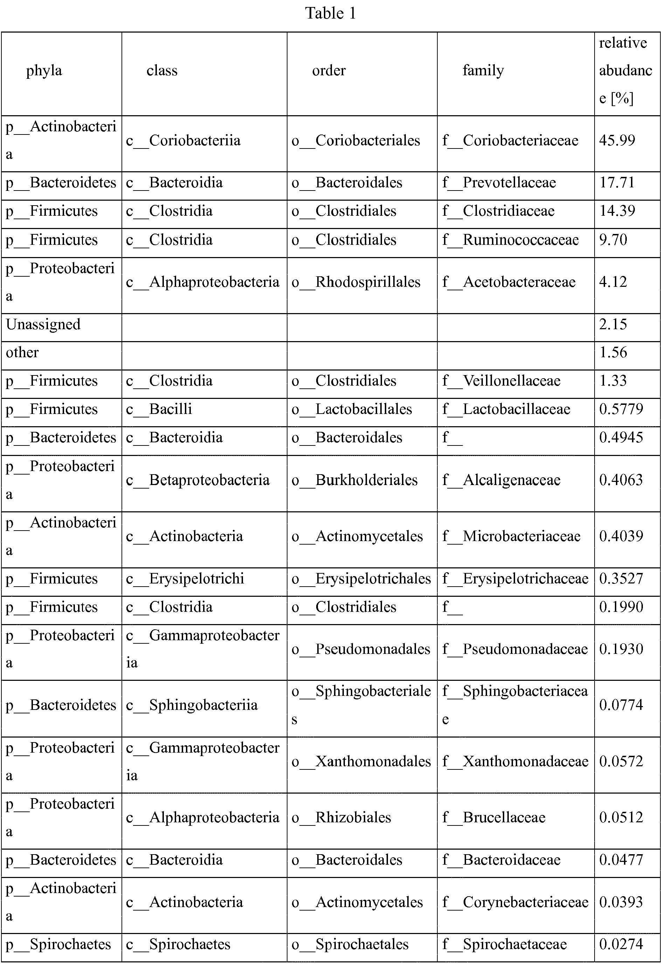

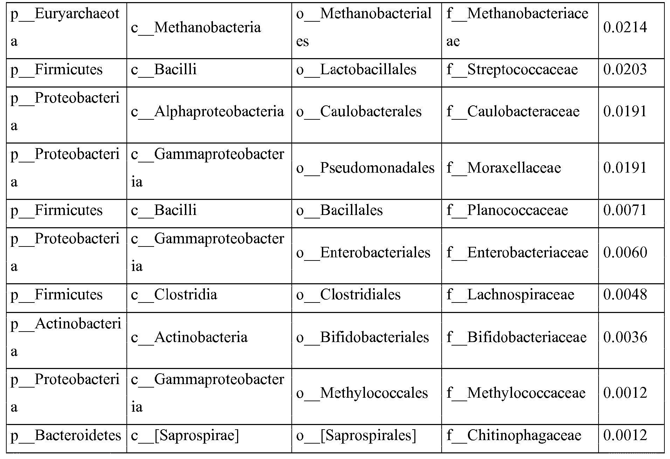

Same conditions as in the example 1 was used but HRT was kept at 2.5 days for the whole operation period. Caproic acid concentration was kept at around 6.5 g/L (fig. 3) (production rate of 2.6 g/L/day). The average caproic acid specificity was 60%. The maximum caproic acid concentration reached 8.5 g/L. Methane formation was inhibited by pH conditions and after 100 days of the process its production dropped to 1.4 mL/L/reactor (methane concentration in the gas stream 0.9%) (fig. 4). Average hydrogen production rate was 20 mL/L/reactor, with maximum at 52 mL/L/reactor (hydrogen concentration in the gas stream 34%) (fig. 4).

## Claims

1. A one-step method for the co-production of caproic acid and hydrogen comprising following steps:
a) providing organic carbon source in the form of biomass and open culture of microorganisms in a single bioreactor, wherein the open culture contains lactic acid producing consortia and consists of at least Coriobacteriaceae, Prevotellaceae, Clostridiaceae, Ruminococcaceae , and Acetobacteraceae families ,
b) transforming the organic carbon source in the presence of the microorganisms, wherein lactic acid, which serves as an electron donor, is produced in-situ as an intermediate product and/or is present in the organic carbon source, and is further converted to caproic acid in the liquid phase and hydrogen in the gaseous phase, wherein the gaseous phase comprises less than 2% of methane and at least 20% of hydrogen;
c) separating formed caproic acid and hydrogen.

2. Method according to claim 1, wherein the transformation of organic carbon source is carried out at 25-40°C, pH of from 4 to 8, organic loading rate between 3.6 and 108 g COD/L/day, and hydraulic retention time between 6 hours and 20 days in a batch, fed-batch or continuous mode.

3. Method according to claim 1 or 2, wherein the open culture of microorganisms are mixed natural consortia of microbes which originate from anaerobic digesters, acidogenic processes, activated sludge, intestinal microorganisms, rumen microorganisms, soil microorganisms, marine microorganisms.

4. Method according to claim 1, 2 or 3, wherein the open culture of microorganisms is supplemented with substrate containing lactic acid mixed consortia, preferably acid whey.

5. Method according to any of previous claims wherein the organic carbon source contains lactic acid.

6. Method according to any of previous claims wherein the biomass is municipal waste and wastewater, agricultural waste and wastewater, waste and wastewater from agro-food industry or lignocellulosic biomass.

## Patentansprüche

1. Einstufiges Verfahren zur gleichzeitigen Herstellung von Capronsäure und Wasserstoff umfassend die folgenden Schritte:
a) Bereitstellung einer organischen Kohlenstoffquelle in Form von Biomasse sowie einer offenen Mikroorganismenkultur in einem einzigen Bioreaktor, wobei die offene Kultur Konsortien von Milchsäure produzierenden Mikroorganismen enthält und mindestens die Familien Coriobacteriaceae, Prevotellaceae, Ruminococcaceae und Acetobacteraceae umfasst,
b) Umwandlung der organischen Kohlenstoffquelle in Gegenwart von Mikroorganismen, wobei Milchsäure, die als Elektronendonor dient, in situ als Zwischenprodukt gebildet wird und/oder in der organischen Kohlenstoffquelle vorhanden ist, und anschließend in der flüssigen Phase zu Capronsäure und in der Gasphase zu Wasserstoff umgewandelt wird, wobei die Gasphase weniger als 2 % Methan und mindestens 20 % Wasserstoff enthält;
c) Abtrennung der entstandenen Capronsäure und des Wasserstoffs.

2. Verfahren nach Anspruch 1, wobei die Umwandlung der organischen Kohlenstoffquelle bei einer Temperatur von 25-40 °C, bei einem pH-Wert von 4 bis 8, bei einer organischen Belastung von 3,6 bis 108 g CSB/l/Tag und einer hydraulischen Verweilzeit von 6 Stunden bis 20 Tagen, im periodischen, periodisch beschickten oder kontinuierlichen Betrieb erfolgt.

3. Verfahren nach Anspruch 1 oder 2, wobei die offene Kultur von Mikroorganismen eine gemischte natürliche Gemeinschaft von Mikroorganismen darstellt, die aus anaeroben Fermentationskammern, acidogenen Prozessen, Belebtschlamm , Darmmikroorganismen, Pansenmikroorganismen, Bodenmikroorganismen oder Meeresmikroorganismen stammen.

4. Verfahren nach Anspruch 1, 2 oder 3, wobei die offene Kultur von Mikroorganismen durch ein Substrat ergänzt wird, das Milchsäure und gemischte Mikroorganismenkonsortien enthält, vorzugsweise saure Molke.

5. Verfahren nach einem der vorstehenden Ansprüche, wobei die organische Kohlenstoffquelle Milchsäure enthält.

6. Verfahren nach einem der vorstehenden Ansprüche, wobei die Biomasse aus Siedlungsabfällen und -abwässern, landwirtschaftlichen Abfällen und Abwässern, Abfällen und Abwässern aus der Agrar- und Lebensmittelindustrie oder lignozellulosehaltiger Biomasse besteht.

## Revendications

1. Procédé en une seule étape pour la coproduction d'acide caproïque et d'hydrogène, comprenant les étapes suivantes:
a) la fourniture d'une source de carbone organique sous forme de biomasse et d'une culture ouverte de micro-organismes dans un bioréacteur unique, dans lequel la culture ouverte contient des consortiums producteurs d'acide lactique et se compose d'au moins les familles des Coriobacteriaceae, Prevotellaceae, Ruminococcaceae et Acetobacteraceae,
b) la transformation de la source de carbone organique en présence des micro-organismes, dans lequel l'acide lactique, qui sert de donneur d'électrons, est produit in situ en tant que produit intermédiaire et/ou est présent dans la source de carbone organique, et ensuite est converti en acide caproïque dans la phase liquide et en hydrogène dans la phase gazeuse, dans lequel la phase gazeuse comprend moins de 2% de méthane et au moins 20% d'hydrogène;
c) la séparation de l'acide caproïque et de l'hydrogène formés.

2. Procédé selon la revendication 1, **caractérisé en ce que** la transformation de la source de carbone organique est effectuée à une température de 25 à 40°C, à un pH de 4 à 8, avec une charge organique volumique comprise entre 3,6 et 108 g DCO/L/jour, et un temps de rétention hydraulique compris entre 6 heures et 20 jours, en mode discontinu (batch), semi-continu (fed-batch) ou continu.

3. Procédé selon la revendication 1 ou 2, **caractérisé en ce que** la culture ouverte de micro-organismes est constituée de consortiums naturels mixtes de microbes qui proviennent de digesteurs anaérobies, de procédés acidogènes, de boues activées, de micro-organismes intestinaux, de micro-organismes du rumen, de micro-organismes du sol, de micro-organismes marins.

4. Procédé selon la revendication 1, 2 ou 3, **caractérisé en ce que** la culture ouverte de micro-organismes est complétée par un substrat contenant d'acide lactique produit par des consortiums mixtes, de préférence du lactosérum acide.

5. Procédé selon l'une quelconque des revendications précédentes, **caractérisé en ce que** la source de carbone organique contient de l'acide lactique.

6. Procédé selon l'une quelconque des revendications précédentes, **caractérisé en ce que** la biomasse consiste en déchets et eaux usées municipaux, déchets et eaux usées agricoles, déchets et eaux usées de l'industrie agroalimentaire ou biomasse lignocellulosique.
